# EUROPEAN PATENT APPLICATION

(11) **EP 2 319 484 A2**
(43) Date of publication of application: **11.05.2011**
(21) Application number: 10163375.8
(22) Date of filing: 20.05.2010
(51) Int. Cl.: A61K 8/06, A61K 8/25, A61K 8/891, A61K 8/893, A61Q 19/00

(54) **Water-in-oil emulsion system and preparation process thereof**

(30) Priority: 03.06.2009 CN 200910146536
(71) Applicant: Evonik Goldschmidt GmbH, 45127 Essen (DE)
(72) Inventor: Zhang, Haizhou, 201108, Shanghai (CN); Zou, Jiali, 201108, Shanghai (CN); Dai, Jingya, 201108, Shanghai (CN); Gao, Zhiheng, 201108, Shanghai (CN)

(57) **Abstract**

The present invention relates to a water-in-oil system, which comprises 1,5-9 wt.% of hydrophobic silica, 0,1-5 wt.% of a water-in-oil emulsifier, 10-40 wt.% of an oil phase, with the content of the oil phase not including the hydrophobic silica and water-in-oil emulsifier therein, and 55-85 wt.% of a water phase; with the proviso that said hydrophobic silica is not AEROSIL® RY200 hydrophobic fumed silica, and it is excluded the formulation comprising the components of: polyoxyalkylene modified polysiloxane SH3775E, a compound of the following formula (I) and hydrophobic fumed silica AEROSIL® R812, in which the weight ratio of SH3775E to the compound of formula (I) is 0,5, and the weight ratio of the sum of SH3775E and the compound of formula (I) to hydrophobic fumed silica AEROSIL® R812 is also 0,5. The weight ratio of the hydrophobic silica to the oil-in-water emulsifier is preferably in a range between 0,7α to 1,2α, in which α is the optimum ratio of the hydrophobic silica to the oil-in-water emulsifier. The present invention also relates to the preparation of this oil-in-water emulsion system. This emulsion system has good "water-releasing effects", and can be widely used in the cosmetic industry

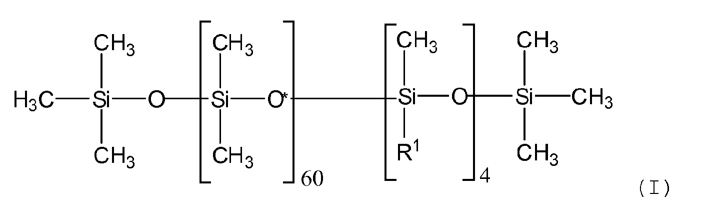

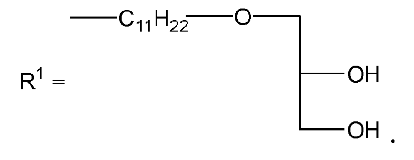

## Description

### Technical Field

The present invention relates to a stable water-in-oil emulsion system and a preparation process thereof. This emulsion system has a "water-releasing effect", and can be widely used in the cosmetic industry.

### Prior Art

CN87107781A discloses a stable composition for local skin use, and it relates to an oil-in-water emulsion containing a modified polymer of acrylic acid series. When the emulsion contacts an electrolyte or skin, it can demulsify so as to coalesce instantly, and to release oil components from the emulsion.

CN1223572A discloses a cleansing product of an inverse emulsion containing a polarphobic region and a high internal phase. The emulsion contained in the cleansing product comprises a continuous external lipid phase and a dispersed internal polar phase. The continuous lipid phase in the emulsion is very fragile, and it breaks when it is subjected to a shearing force during use, and thereby releases the dispersed internal phase.

As to the products produced by the above two technologies, although they can also release a water phase during their contact with skin or in the low shearing and applying process, resulting in a moisturizing and soothing experience, both of them cannot quickly generate a large amount of bead-like water drops on the skin surface. There is another product of a water-in-oil emulsion system which can release the water phase during a normal application process, and can instantly produce a large amount of bead-like water drops on the skin and bring about a novel and soothing feeling to the consumers, and therefore it is popular with the consumers (Asia Pacific PERSONAL CARE,issue 2, volume 9, March, 2008, page 45 ). In the present invention, this product which produces a large amount of bead-like water drops when applied onto the skin surface is referred to as "water-releasing cream" for short; and this effect of producing a large amount of bead-like water drops when applied on the skin surface is referred to as "water-releasing effect". Such water-releasing cream products are rarely seen on the market, since these products are realized by an ultra high ratio of the internal phase (water phase). Usually, in order to achieve quite ideal a water-releasing effect, it is often required that the amount of the internal phase is at least more than 85 wt.% of the total amount, that is to say, it needs to use a continuous phase of less than 15 wt.% to coat a dispersed water phase of more than 85 wt.%.

There are the following disadvantages existent in the above product having an ultra high content of the internal phase: firstly, it requires a very high emulsion capability by the emulsifier, in other words, there are certain restrictions to the use of emulsifiers. For example, only the emulsifiers of a relatively high emulsion efficiency, such as alkyl-co-polysiloxane, can be used. Secondly, in view of the preparation process, since the content of the internal phase is large, when it reaches the later stage by adding the internal phase gradually, the viscosity of the system would already be very high, and it would often be difficult for the remaining internal phase to be added therein, then it often takes a long time to add the complete internal phase, the technical control for the whole process would be very sensitive, and the failure rate during the industrialized production would be quite high. Thirdly, since the content of the internal phase (water phase) is very high, the emulsified particles have to be very large, otherwise the viscosity would be too high to operate; however, large emulsified particles would lead to high sensitivity to shearing, and it would be difficult to ensure the long term stability of the formulation, and therefore it would not be easy to actually carry out large scale production and commercialization. Fourthly, since the ratio of the external phase (oil phase) is very low, the selection of oils and fats would be more restrictive, and it is very difficult for such a formulation to form other functional products, such as sunscreen products with a relatively high SPF value or products such as foundation make up creams. Fifthly, also due to the influence of the ultra high content of the internal phase, the emulsion particles have to be very large, and the appearance of the whole products becomes relatively rough, thus they cannot form a bright and aesthetically appealing appearance.

If a water-releasing cream can be found which is capable of providing good water-releasing effects at an conventional content of the internal phase such as 70 wt.%, 60 wt.%, and even 55 wt.%, then the operability of the formulation would be greatly enhanced, and the extension of its functionalities would also be achieved easily. However, no such products currently exist.

### Description of the invention

In view of the above-mentioned status of the prior art an object of the present invention is to provide a water-in-oil emulsion system, **characterized in that** this emulsion system can achieve a good water-releasing effect even at a low content of the internal phase (for example, the percentage of the content of the internal phase in the total amount is as low as 55 wt.%).

Extensive and thorough researches on the "water-releasing cream" products which have water-releasing effects were carried out, and as a result it has been found surprisingly that a product can bear the above-mentioned "water-releasing effect" at a conventional internal phase content by adding into the water-in-oil emulsion system a certain amount of hydrophobic silica, and especially hydrophobic fumed silica, and by controlling the weight ratio of the hydrophobic silica to the water-in-oil emulsifier within a specified ratio range.

An advantage of the instant invention is, since the amount of the internal phase is reduced, that not only the operation difficulties are greatly reduced thus the time for preparing the formulation is greatly shortened, but also the scope for the selection of the emulsifiers and other oil phase components is broadened at the same time, and this greatly enhances the operability and variability of the formulations.

The above object is achieved by a water-in-oil emulsion system, which water-in-oil system comprises, on the basis of the total weight of the emulsion system, 1,5-9 wt.% of hydrophobic silica, 0,1-5 wt.% of a water-in-oil emulsifier, 10-40 wt.% of an oil phase, with the content of the oil phase not including said hydrophobic silica and water-in-oil emulsifier, and 55-85% wt.% of a water phase, in which the weight ratio of the hydrophobic silica to the oil-in-water emulsifier is preferably in a range between 0,7α to 1,2α, in which α is an optimum ratio of the hydrophobic silica to the oil-in-water emulsifier (also referred to as a characteristic ratio α), and is obtained by the process described below.

The above object can also be achieved by such a water-in-oil emulsion system that the water-in-oil system comprises, on the basis of the total weight of the emulsion system, 1,5-9 wt.% of hydrophobic silica, 0,1-5 wt.% of a water-in-oil emulsifier, 10-40 wt.% of an oil phase, with the oil phase content not including said hydrophobic silica and water-in-oil emulsifier, and 55-85% wt.% of a water phase, in which the water-in-oil emulsion system has a good water-releasing effect described herein below in the present invention.

It must be pointed out that, the hydrophobic silica which can be used in the present invention does not include AEROSIL® RY200 hydrophobic fumed silica (produced by AEROSIL company, Japan), and also excluded are the formulations comprising the following components: polyoxyalkylene modified polysiloxane SH3775E (produced by Toray Silicone company), alkyl glyceryl ether modified polysiloxane of the following formula (I) and hydrophobic fumed silica AEROSIL® R812 (produced by Evonik Degussa GmbH), in which the weight ratio of SH3775E to said alkyl glyceryl ether modified polysiloxane is 0,5, and the weight ratio of the sum of SH3775E and said alkyl glyceryl ether modified polysiloxane to said hydrophobic fumed silica AEROSIL® R812 is also 0,5, 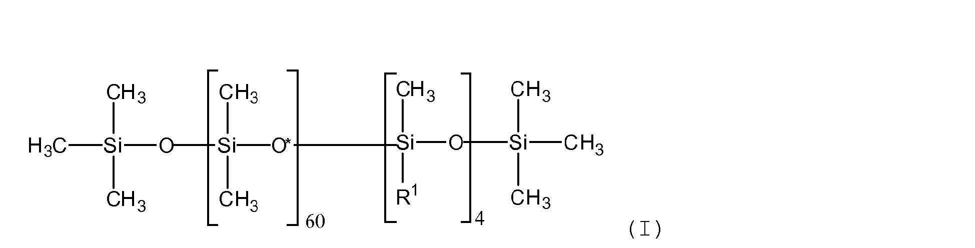 in which

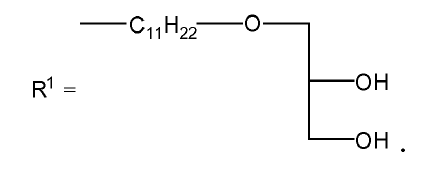

The water-in-oil emulsion system of the present invention is of a milk-white, frosty and bright appearance, or it can change into a transparent gel-like or matt appearance by a method of adjusting the refractive index according to requirements; at the same time, when applied to the skin in a conventional way, it quickly releases bead-like water drops, which brings about a novel and soothing feeling to the consumers. Such a formulation can be used in various fields of skincare, and in products for hair care and colour cosmetics.

With respect to the present invention, a good water-releasing effect of the water-in-oil system means that the water-releasing effect has an evaluation result of more than or equal to a score of 3 in the evaluation system described below. The test method and evaluation score of the test system are described below.

About 0,2 g of a water-in-oil emulsion sample is taken and placed on the back of a hand, then it is applied thereon by circling gently with the middle finger and ring finger of the other hand, and then the phenomenon of the water-releasing effect is observed when the circling application reaches 20 cycles, and evaluated by a 5-level scoring system. Score 5 represents that more than 10 bead-like water drops having an average diameter of more than or equal to 3 mm appear, or more than 20 bead-like water drops having an average diameter of more than or equal to 1 mm appear; score 4 represents that 2-10 bead-like water drops having an average diameter of more than or equal to 3 mm appear, or 10-20 bead-like water drops having an average diameter of more than or equal to 1 mm appear and the bead-like water drops having an average of more than or equal to 3 mm are no more than 10; score 3 represents that 2-9 bead-like water drops having an average diameter of more than or equal to 1 mm appear and there is at most 1 bead-like water drop having an average diameter of more than or equal to 3 mm, or 10-20 bead-like water drops having an average diameter of 1 mm appear; score 2 represents that 2-9 bead-like water drops having an average diameter of 1 mm appear; and score 1 represents that no water drop appears. Each level between scores 5 to 4, 4 to 3, 3 to 2, and 2 to 1 shows that the water-releasing effect is between the two end values described above, and the lower the score, the poorer the water-releasing effect.

In a preferred embodiment of the water-in-oil emulsion system in the present invention, the water-in-oil emulsion system comprises, on the basis of the total weight of the emulsion system, 1,5-5 wt.% of the hydrophobic silica, 0,3-2,5 wt.% of the water-in-oil emulsifier, 15-40 wt.% of the oil phase, with the oil phase content not including the hydrophobic silica and the water-in-oil emulsifier, and 55-80 wt.% of the water phase.

According to the present invention, it is preferred that, in the water-in-oil emulsion system, hydrophobic silica which accounts for a relatively high proportion in the oil phase, especially hydrophobic fumed silica (sometimes also referred to as "pyrogenic silica" in the present invention as it is made from flame pyrolysis). It is advantageous to the present invention that, in order to obtain an optimized water-releasing effects, the amount of the hydrophobic silica (especially the hydrophobic fumed silica) accounts for 5-15 wt.% of the weight of the oil phase, preferably 5,5-15 wt.%, more preferably 5,5-13 wt.%, and particularly preferably 7-13 wt.%, with the oil phase here not including the hydrophobic silica and the water-in-oil emulsifier.

The hydrophobic silica used in the present invention, also referred to as hydrophobic silica, comprises hydrophobic fumed silica and hydrophobic precipitation-process silica, among which the hydrophobic silica produced by a gaseous phase process is preferred. After having been substituted by alkyl groups, the silica products are classified according to the different substitution groups into silylated silica, dimethyl-silylated silica, trimethyl-silylated silica and polydimethylsiloxane-silylated silica. The hydrophobic silica especially suited for the present invention are those having a methanol wettability (hydrophobicity) of 10-85%, and preferably 25-75%, and having a BET surface area of 70-350 m²/g, and preferably 100-280 m²/g_{.}

The hydrophobic fumed silica includes the hydrophobic fumed silica supplied under the trade name of AEROSIL®, Cab-o-Sil® and HDK®, and is preferably one or more selected from the group consisting of AEROSIL® R202, AEROSIL® R972, AEROSIL® R805, AEROSIL® R8200, AEROSIL® R974, AEROSIL® R812S and AEROSIL® R812 (all supplied by Evonik Degussa GmbH). The hydrophobic precipitation silica suitable for the present invention includes SIPERNAT® D17 precipitation silica (supplied by Evonik Degussa GmbH).

For the measurement method of methanol wettability reference is made to the corresponding parts in the descriptions of US6899951 and CN1264933C.

In the water-in-oil emulsion system according to the present invention, a water-in-oil emulsifier is used, and the water-in-oil emulsifier is those conventionally used in water-in-oil emulsions. The emulsifier is preferably a surfactant having an HLB value of 2-8, and is selected from at least one of the following three types (in which the hydrophobic group is a polyether glycol group, preferably polyoxyethenyl, polyoxypropenyl, polyglyceryl, or a polyoxyethylene sorbitan group):
the first type: an emulsifier having a molecular chain structure of polysiloxane + a hydrophilic group + an alkyl, which comprises a block copolymer emulsifier of polysiloxane, a polyetherpolyol and an aliphatic alkane connected by covalent bonds, an emulsifier with a polysiloxane chain as the main chain, and polyetherpolyol and aliphatic alkyl as side groups which are respectively connected to the main chain of polysiloxane by covalent bonds, and an emulsifier with a polysiloxane chain as the main chain, aliphatic alkyl modified polyetherpolyol as side groups which are connected to the polysiloxane chain by covalent bonds; preferably C6-C20 alkyl-copolymerized polyoxyethene polydimethylsiloxane, and more preferably cetyl PEG/PPG-10/1 polydimethylsiloxane (supplied under the trade name of ABIL® EM90 by Evonik Degussa);
the second type: an emulsifier having a molecular chain structure of polysiloxane + a hydrophilic group, which emulsifier comprises a block copolymer emulsifier with polysiloxane and a polyetherpolyol connected by covalent bonds, an emulsifier with a polysiloxane chain as the main chain, and polyetherpolyol as side groups connected to the main chain of polysiloxane by covalent bonds; preferably polyoxyethylene polydimethylsiloxane, polyglycerol polydimethylsiloxane, and more preferably bis-PEG/PPG-14/14 polydimethylsiloxane (supplied under the trade name of ABIL® EM97 by Evonik Degussa GmbH); and
the third type: an ester emulsifier formed by a polyetherpolyol of a non-linear structure or a linear structure, via its hydroxyl group, with a fatty acid, or an ether emulsifier formed by a polyetherpolyol, via its hydroxyl group, with a fatty alcohol, which comprises a polyoxyethylene fatty acid ester, a polyoxyethylene alkyl ether, a mono- or polyglycerol fatty acid ester, a mono- or polyglycerol alkyl ether, preferably diisostearyl polyglyceryl-3dimer dilinoleate (supplied under the trade name of ISOLAN® PDI by Evonik Degussa GmbH), polyglyceryl-4 diisostearate/polyhydroxystearate/sebacate (supplied under the trade name ISOLAN® GPS by Evonik Degussa GmbH) and a polyoxyethylene sorbitan anhydride fatty acid ester. According to the present invention, it is preferred that in the present invention the amount of the water-in-oil emulsifier used is adjusted according to a certain ratio based on the amount of hydrophobic fumed silica used. Taking the emulsifiers ABIL® EM90 (as the water-in-oil emulsifier) and silylated silica AEROSIL® R812S (as the hydrophobic fumed silica) as examples, AEROSIL® R812S is first defined at a percentage between 7-13wt.% of the oil phase (with the oil phase not including the hydrophobic silica and the emulsifier), by gradually adjusting the amount of ABIL® EM90 used, it can be found that when the weight ratio of hydrophobic fumed silica AEROSIL® R812S to the emulsifier ABIL® EM90 is between 3-6, and particularly when it is close to 4,3, the better the water-releasing effect of the water-in-oil emulsion product obtained will be (see embodiment 1). If it is above the ratio of 6 (1,4α), the emulsion process becomes apparently difficult and cannot form a stable emulsion system, while when it is below 3 (0,7α), only a small amount of water drops (the water-releasing effect is score 2 or even lower) can be formed, which cannot reach the standard of a good water-releasing effect of the present invention, and moisturizing skin feel is not apparent.

Then taking the emulsifier ISOLAN® PDI and silylated silica AEROSIL® R812S as examples (embodiment 13), AEROSIL® R812S is defined at 3 wt.% of the emulsion system, by gradually adjusting the amount of ISOLAN® PDI used, it can be found that when the weight ratio of the hydrophobic fumed silica AEROSIL® R812S to the emulsifier ISOLAN® PDI is between 8,4-15, and particularly when it is close to 12, the better the water-releasing effect will be. If it is above the ratio of 15 (1,25α), the emulsion process becomes apparently difficult and cannot form a stable emulsion system, while when it is below 8,4 (0,7α), only a small number of water drops (the water-releasing effect is score 2 of even lower) are formed, which cannot reach the standard of a good water-releasing effect according to the present invention, and moisturizing skin feel is not apparent.

In the present invention, the optimum weight ratio of the hydrophobic silica to the water-in-oil emulsifier is referred to as a characteristic ratio α, and the characteristic α can be detemined by the following process:
(i) providing the weight parts of the hydrophobic silica, the oil phase and the water phase in the water-in-oil emulsion respectively as weight parts of X, M, and Z, wherein the oil phase does not include hydrophobic silica and the water-in-oil emulsifier;
(ii) mixing uniformly X weight parts of the hydrophobic silica, M weight parts of the oil phase, Y₀ weight parts of the water-in-oil system, so as to obtain an oil phase mixture A;
(iii) adding slowly under stirring Z₀ weight parts of the water phase into the oil phase mixture A, so as to make the system into a uniformly emulsified state;
(iv) adding continuously Z₁ weight parts of the water phase, until visually observing that the water phase can no longer be emulsified and, then adding Y₁ weight parts of the water-in-oil emulsifier to the emulsion system under stirring, so as to have the system emulsified completely;
(v) repeating step (iv) n times by using Zₙ₊₁ weight parts of the water phase and Yₙ₊₁ weight parts of the water-in-oil emulsifier, until all of Z weight parts of the water phase have been added into the oil phase mixture A, thus obtaining a uniform water-in-oil emulsion system C, wherein Z=Z₀+Z₁+.....+Zₙ₊₁, and wherein n is a natural number and n>1; and
(vi) calculating the total weight Y of the water-in-oil emulsifier used in the emulsion system C, wherein Y = Y₀+Y₁+.....+Y₀₊₁, thereby obtaining the optimum weight ratio X/Y of the hydrophobic silica to the water-in-oil emulsifier, i.e. the characteristic ratio α, in which n is a natural number, and n>1.

In the above-mentioned process for determining the characteristic ratio α, X weight parts of the hydrophobic silica should be made such that the content of the X weight parts hydrophobic silica is 1,5-9 wt.%, and preferably 1,5-5 wt.%, based on the total weight of the water-in-oil emulsion system.

In the above-mentioned process for determining the characteristic ratio α, Y₀, Y₁ and Yₙ₊₁ weight parts of the water-in-oil emulsifier can be either the same or different in terms of weight, and each of them is made such that its content is 0,01-0,3 wt.%, and preferably 0,05-0,2 wt.%, based on the total weight of the water-in-oil emulsion system.

In the above-mentioned process for determining the characteristic ratio α, Z₀, Z₁ ...... Zₙ₊₁ weight parts of the water phase can be either the same or different in terms of weight, and each of them is made such that its content is 2-9 wt.%, and preferably 4-9 wt.%, based on the total weight of the water-in-oil emulsion system

In a particularly preferred embodiment of the present invention, the following combinations of the hydrophobic silica and the water-in-oil emulsifier are used, and the preferred characteristic ratios α of these combinations are shown in the table below:

| characteristic α | ABIL® EM90 | ISOLAN® PDI | ISOLAN® GPS | ABIL® EM97 |
|---|---|---|---|---|
| AEROSIL® R812S | 4,3 | 12 | 15 | 3 |
| AEROSIL® R805 | 2,3 | 15 | 6 | 2,6 |
| AEROSIL® R974 | 2,3 | 7,5 | 5,5 | 1,5 |
| AEROSIL® R972 | 4,3 | 12 | 12 | 2,7 |
| AEROSIL® R202 | 20 | 15 | 30 | 7,5 |
| SIPERNAT® D17 | 7,5 | | | |

Most of the hydrophobic silica products have the effect of thickening the oil phase, and in the ordinary water-in-oil cream emulsions, hydrophobic silica generally accounts for 0,01-1 wt.% of the total amount of the emulsion. However, in the water-in-oil emulsion system of the present invention, the proportion that the hydrophobic silica accounts for the total weight of the emulsion system is more than the amount of a common thickener used, and reaches 1,5-9wt.%, and preferably 1,5-5wt.%. Although at the moment there is no clear mechanism for this, the inventor considers that in this case the hydrophobic silica may competitively occupy the interface of the emulsion particles, and after the ratio of hydrophobic silica to the emulsifier having been increased to a certain extent (0,7α), the emulsion particles will release the internal phase easily when being in such a grinding manner of "applying" , thus providing the "water-releasing" effect. When the ratio of the hydrophobic silica to the emulsifier is further increased, the water-releasing effect will be further enhanced, but once it is beyond a specific ratio (1,2 α), the emulsifier will no longer be able to stay on the interface thus it cannot reduce the interface tension effectively, and hence it cannot emulsify effectively. Since the hydrophobic silica can itself thicken an oil phase and form a gel network in the external phase, the hydrophobic silica has an obvious aiding effect on the stability of the system. However, due to the competition of the hydrophobic silica and the emulsifier in a specific ratio range, the hydrophobic silica and the emulsifier are made to have a specific relationship of both competition and synergy, therefore, if the ratio of the hydrophobic silica and the emulsifier does not exceed the specific ratio range, which is between 0,7 α to 1,2 α, the system will not be sensitive to a conventional shearing, such as stirring and homogenization, and releases the internal phase only under rolling-pressing.

In the present invention, the amount of the hydrophobic silica used can be adjusted according to the viscosity of the water-in-oil emulsion system and the content of the oil phase. Generally speaking, the viscosity of the system can be increased by increasing the amount of the hydrophobic silica used; and a relatively high content of oil phase also needs a high content of hydrophobic silica. When the viscosity of the oil components in the oil phase is relatively high, the amount of hydrophobic silica used can be reduced by an appropriate amount. For example, if diethylhexyl carbonate (TEGO®SOFT DEC) of a relatively high viscosity is used to replace cyclic methyl siloxane of a relatively low viscosity, and in this case, the water-releasing effect of the obtained product will still be very good (Table 6) by properly reducing the content of hydrophobic silica (reduced from 3 wt.% to 1,5 wt.%), and by adjusting the amount of the emulsifier ABIL® EM 90 according to a characteristic ratio α (the characteristic ratio α of AEROSIL® R 974 to ABIL® EM 90 being between 2,1 to 2,3).

According to the actual test results, if the hydrophobic silica is not used, or it is used only at a conventional amount for hydrophobic silica, for example below 0,5 wt.%, the water content in the internal phase needs to be up to 85 wt.% to have a significant water-releasing effect (see comparative example 1 in Table 1), while the change of the amount of the emulsifier used has no significant influence to the water-releasing effect.

In the water-in-oil emulsion system of the present invention, the oil phase is any oil phase conventionally contained in a water-in-oil emulsion; there is no other particular restriction to the selection of each oil phase component in the oil phase, and all oil phase components which are allowed to be used in cosmetics can be used here. It can be at least one oil/fat selected from vegetable oil, mineral oil, silicon oil and synthesized oil that are traditionally used, and can also be various waxes that are traditionally used.

The oil/fat such as silicon oil suitable for the present invention is, for example, polydimethylsiloxane and cyclic methylsiloxane, and also aryl- or alkyl- or alkoxy-substituted polymethylsiloxanes or cyclic methylsiloxanes. The oil/fat suitable for the present invention also comprises mono- or diesters of linear and/or branched mono- and/or dicarboxylic acids having 2 to 44 carbon atoms with saturated or unsaturated, linear and/or branched alcohols having 1 to 22 carbon atoms. Likewise, as the oil/fat used in the present invention, use can also be made of the esters of bifunctional aliphatic alcohols having 2-36 carbon atoms with monofunctional aliphatic carboxylic acids having 1-22 carbon atoms.

As the oil/fat used in the present invention, use may also be made particularly of esters of fatty acids having 12-22 carbon atoms, such as methyl esters and isopropyl esters, such as methyl laurate, methyl stearate, methyl oleate, methyl erucate, isopropyl palmitate, isopropyl myristate, isopropyl stearate and/or isopropyl oleate.

In addition, as the oil/fat used in the present invention, preference is particularly also given to n-butyl stearate, n-hexyl laurate, n-decyl oleate, isooctyl stearate, isononyl palmitate, isononyl isononanoate, 2-ethylhexyl palmitate, 2-ethylhexyl laurate, 2 hexyl-decyl stearate, 2-octyldodecyl palmitate, oleyl oleate, oleyl erucate and/or erucyl oleate.

As the oil/fat used in the present invention, dicarboxylic acid esters are also especially suitable, such as di(n-butyl) adipate, di(n-butyl) sebacate, di(2-ethylhexyl) adipate, di(2-hexyldecyl) succinate and/or diiso-tridecyl azelate. As the oil/fat used in the present invention, diol esters are also especially suitable, such as ethylene glycol dioleate, ethylene glycol diisotri-decanoate, propylene glycol di(2-ethylhexanoate), butanediol diisostearate and/or neopentyl glycol dicaprylate.

As the oil/fat used in the present invention, use may also be made of carbolic acid diesters, such as diethylhexyl carbonate.

It is also applicable to use relatively long-chain triglycerides, i.e. triple esters of glycerol with three acid molecules, at least one of which is a relatively long-chain acid molecule. Mention may be made here, by way of example, of fatty acid triglycerides, which comprise synthetic triglycerides of caprylic/capric acid mixtures, triglycerides of industrial oleic acid, triglycerides of isostearic acid and triglycerides of palmitic/oleic acid mixtures. Use may additionally be made of linear or branched fatty alcohols, such as oleyl alcohol or octyldodecanol, and also fatty alcohol ethers, such as dioctyl ether, PPG-3 myristyl ether, etc.

As the oil/fat used in the present invention, it is also applicable to use, for example, natural vegetable oils, e.g. olive oil, sunflower oil, soybean oil, peanut oil, rapeseed oil, almond oil or palm oil or jojoban oil, and also the liquid portion of coconut oil or palm kernel oil, and also the liquid portions of animal oils, such as sperm oil, neatsfoot oil or beef tallow.

As the oil/fat of the present invention, use may further be made of hydrocarbon oils, particularly liquid paraffin and isoparaffin. Examples of hydrocarbon oil/fat which can be used are paraffin oil, white mineral oil, isohexadecane, polydecene, petroleum jelly, light liquid paraffin or squalane. Furthermore, esters of arylcarboxylic acids are also suitable, such as, esters of benzoic acid, e.g. benzoic acid esters formed by the esterification of saturated or unsaturated and linear or branched alcohols having 1-22 carbon atoms with benzoic acid, such as isostearyl benzoate and octyldodecyl benzoate, preferably C12-15 alkyl benzoate.

In a preferred embodiment of the present invention, the components of the oil/fat preferably comprise one or more selected from the group consisting of cetyl polydimethylsiloxane (ABIL® WAX9801), diethylhexyl carbonate (TEGO® SOFT DEC), cyclic methylsiloxane, polydimethylsiloxane (ABIL® 350), white mineral oil and ABIL® OSW5 cyclic methylsiloxane/ dimethylsilanol etc.

The content of each component in the oil phase is also the common amount used in the water-in-oil emulsion, with no particular restriction in the present invention.

In the water-in-oil emulsion system of the present invention, the water phase forms a disperse phase of the water-in-oil emulsion. The water phase can only comprise therein water, or comprises other substances other than water which can be dissolved in water. Such substances can be those substances commonly contained in the water phase with respect to a water-in-oil emulsion of a preparation for personal care and make-up. This especially comprises ethanol and/or a C2-C5 polyol containing two or more hydroxyl groups (such as three hydroxyl groups), and the latter is preferably glycerol, propanediol, 1,3-butanediol or any mixture thereof, and especially glycerol. In addition, the water phase can comprises a water soluble polymer, which water soluble polymer is selected from one or more of the following group of xanthan gum, guar gum, chondroitin sodium sulfate, sodium hyaluronate, arabic gum, sodium alginate, carrageenan, hydroxypropyl cellulose, methylcellulose, substituted methylcellulose (such as hydroxypropylmethyl cellulose), polyacrylic acid, and polyacrylic acid with an alkyl substituent on the main chain (such as TEGO® Carbomer 341 ER). The water soluble polymer is especially xanthan gum.

Of course, those skilled in the art will understand that, besides the above-mentioned components, in the water-in-oil emulsion system of the present invention, other auxiliary components can also be contained, such as a humectant, an emollient, a free radical scavenger, a chelator, an antioxidant, an essence, a preservative (such as 2-bromo-2-nitro-1,3-propanediol), a film-former, a stabilizer (such as sodium chloride, magnesium chloride and magnesium sulfate) etc. These auxiliary components are comprised in the oil phase or water phase, depending on their solubility in the water phase and oil phase.

In the water-in-oil emulsion system of the present invention, any liposoluble or lipo-dispersible component, such as sun screening agent and hydrophobic modified powder, can also be added therein.

As UV screening agents, sun screening agents, for example, can be organic substances, they absorb ultraviolet radiation and radiate the energy absorbed in the form of a longer wavelength, for example, heat.

UV-B screening agent can be oil soluble or water soluble; if an oil soluble UV-B screening agent is used, it is comprised in the oil phase of the emulsion in the present invention; and if a water soluble UV-B screening agent is used, it is comprised in the water phase of the emulsion in the present invention.

As an oil soluble UV screening agent, reference can be made, for example, of:
3-benzylidenecamphor and derivatives thereof, such as 3-(4-methylbenzylidene)camphor;
4-aminobenzoic acid derivatives, such as 2-ethylhexyl 4-(dimethylamino)benzoate and pentyl 4-(dimethylamino)benzoate;
cinnamates, such as 2-ethylhexyl 4-methoxyl cinnamate, isopentyl 4-methoxyl cinnamate, and 2-ethylhexyl 2-cyano-2-phenyl cinnamate (octocrylene);
salicylates, such as 2-ethylhexyl salicylate, 4-isopropylbenzyl salicylate and menthyl salicylate;
benzophenone derivatives, such as 2-hydroxy-4-methoxybenzophenone, 2-hydroxy-4-methoxy-4'-methyl benzophenone and 2,2'-dihydroxy-4-methoxybenzophenone; benzylidene malonates, such as di(2-ethylhexyl) 4-methoxybenzylidene malonate;
triazine derivatives, such as 2,4,6-trianilino-(p-carbo-2'-ethyl-1'-hexyloxy)-1,3,5-triazine and octyltriazone; and propane-1,3-diones, such as 1-(4-tert-butylphenyl)-3-(4'-methoxyphenyl)propane-1,3-dione.

The water soluble UV-B screening agent that can be used is:
2-phenylbenzimidazole-5-sulfonic acid, and its alkaline metal, alkaline earth metal, ammonium, alkyl ammonium, alkanol ammonium and glucose ammonium salts;
benzophenone sulfonic acid derivatives and their salts, such as 2-hydroxy-4-methoxybenzophenone-5-sulfonic acid and its slats;
3-benzylidene camphor sulfonic acid derivatives and salts thereof, such as 4-(2-oxo-3-norbornylene-methyl)benzenesulfonic acid, 2-methyl-5-(2-oxo-3-norbornylene)benzenesulfonic acid and salts thereof.

Benzoyl methane derivatives can particularly be used as typical UV-A screening agents, such as 1-(4'-(tert-butyl)phenyl)-3-(4'-metoxyphenyl)propane-1,3-dione and 1-phenyl-3-(4'-isopropylphenyl)propane-1,3-dione. It is obvious that the UV-A and UV-B screening agents can be used in mixture.

In addition to the soluble screening agent substances mentioned, insoluble pigments, i.e. finely divided metal oxides or salts can also be used in the present invention, such as titanium dioxide, zinc oxide, iron oxide, aluminium oxide, cerium oxide, zirconium oxide, silicates (talcum), barium sulfate, and zinc stearate. Those relating to this are that the average particle size thereof should be less than 100 nm, such as between 5-50 nm, and particularly between 15-30 nm. They can be in a spherical shape; however, those of a elliptical shape, or of a shape derived from a spherical shape in another way can also be used. A class of relatively new screening agents comprise micronized organic pigments, such as 2,2'-methylenebis[6-(2H-benzotriazole-2-yl)-4-(1,1,3,3-tetramethylbutyl)phenol], which has a particle size of less than 200 nm, and for example it can be obtained as 50wt% of an aqueous dispersion.

In addition, other suitable UV screening agents can be found in the review of SÖFW-journal, volume 122, page 543 (1996) by P. Finkel.

In addition to the two groups of primary UV/light screening agents mentioned above, secondary light screening agents of the antioxidants type can also be used, they interrupt the photochemical reaction chain which is triggered if UV radiation penetrates the skin. As antioxidants, use can be made, for example, of superoxide dismutases, tocopherol (Vitamin E), 2,6-dihydroxylbutyltoluene, and ascorbic acid (Vitamin C).

As the solids, use can be made, for example, of iron oxide pigment, titanium dioxide or zinc oxide particles. In addition, particles which results in special sensory effects can be used, such as nylon-12, boron nitride, polymer particles, such as polyacrylate or polymethacrylate particles or organosilicon elastomers.

As pearlescent addictives, use can be made, for example, of ethylene glycol distearate and/or PEG-3 distearate.

As insectifuges, use can be made, for example, of N,N-diethyl-m-toluamide, 1,2-pentanediol and/or insectifuge 3535.

As suntanning agents, use can be made, for example, of dihydroxyacetone and erythrulose.

As preservatives, use can be made, for example, of a mixture of one or more of alkyl p-hydroxybenzates and phenoxyaethanol. The alkyl p-hydroxybenzates can be methyl p-hydroxybenzate, ethyl p-hydroxybenzate, propyl p-hydroxybenzate and/or butyl p-hydroxybenzate. Other alcohols can be used instead of phenoxyethanol, such as benzyl alcohol or ethanol. In addition, other conventional preservatives can be used, such as sorbic acid, benzoic acid, salicylic acid, 2-bromo-2-nitropropane-1,3-diol, chloroacetamide, diazoalkylurea, DMDM hydantoin, sodium hydroxylmethylamino acetate, methylisothiazoline, chloromethylisothiazoline, ethylhexyl glycerol and/or caprylyl ethanediol.

Use may be made, as fragrances, of natural or synthetic odoriferous substances or mixtures thereof. Natural odoriferous substances are extracts of flowers (lily, lavender, rose, jasmine, neroli or ylang-ylang), stems and leaves (geranium, patchouli, petitgrain), fruits (anis, coriander, caraway, juniper), fruit rinds (bergamot, lemon, orange), roots (mace, angelica, celery, cardamom, costus, iris, thyme), needles and twigs (spruce, fir, pine, mountain pine) and resins and balsams (galbanum, elemi, benzoin, myrrh, frankincense, opoponax). Animal raw materials are also possible, such as, for example, civet and castoreum. Typical synthetic perfume compounds are products of the esters, ethers, aldehydes, ketones, alcohols and hydrocarbon types. Perfume compounds of the ester type are, e.g., benzyl acetate, phenoxyethyl isobutyrate, p-(tert-butyl)cyclohexyl acetate, linalyl acetate, phenylethyl acetate, linalyl benzoate, benzyl formate, methyl phenylglycinate, allylcyclohexyl propionate, styrallyl propionate and benzyl salicylate; perfume compounds of ethers include, for example, benzyl ethyl ether; perfume compounds of aldehydes include, e.g., linear alkanals having 8 to 18 carbon atoms, citral, citronellal, citronellyloxy-acetaldehyde, cyclamen aldehyde, hydroxycitronellal, lilial and bourgeonal; perfume compounds of ketones include, e.g., the ionones, alpha isomethyl ionone and methyl cedryl ketone; perfume compounds of alcohols include anethole, citronellol, eugenol, isoeugenol, geraniol, linalool, phenylethyl alcohol and terpineol; and perfume compounds of hydrocarbons include mainly the terpenes and balsams. Use may be made of mixtures of different odoriferous substances which together generate an attractive scent. Essential oils of low volatility, which are generally used as flavouring components, are also suitable as fragrances, e.g. sage oil, camomile oil, clove oil, balm oil, peppermint oil, cinnamon leaf oil, linden blossom oil, juniper berry oil, vetiver oil, frankincense oil, galbanum oil, labdanum oil and lavandin oil. Use may be made of bergamot oil, dihydromyrcenol, lilial, lyral, citronellol, phenylethyl alcohol, α-hexyl-cinnamaldehyde, geraniol, benzylacetone, cyclamen aldehyde, linalool, Boisambrene Forte, Ambroxan, indole, Hedione, lemon oil, mandarin oil, orange oil, allyl amyl glycolate, lavandin oil, sage oil, damascone, geranium oil, cyclohexyl salicylate, Vertofix Coeur, phenylacetic acid, geranyl acetate, benzyl acetate, rose oxide, alone or in mixtures.

As colorants, the substances suitable and having been authorized for use in cosmetics are, for example, those compiled in the publication "Kosmetische Färbemittel" [Cosmetic Colouring Agents] of the Farbstoffkommission der Deutschen Forschungsgemeinschaft [Colorant Commission of the German Research Association], Verlag Chemie, Weinheim, 1984, pp. 81-106. These colorants are only used in the concentration of 0,001-0,1% by weight, based on the entire mixture.

In the water-in-oil emulsion system of the present invention, a conventional amount of biogenic active substance can also be added. The term "biogenic active substances" should be understood to mean, for example, tocopherol and derivatives thereof, ascorbic acids and derivatives thereof, retinol and derivatives thereof, deoxyribonucleic acid, coenzyme Q10, bisabolol, allantoin, phytantriol, panthenol, beta-hydroxy acids, salicylic acid, amino acids and derivatives thereof, hyaluronic acid, glucans, creatine and derivatives thereof, guanidine and derivatives thereof, ceramides, phytosphingosine and derivatives thereof, sphingosine and derivatives thereof, pseudoceramides, essential oils, peptides, protein hydrolysates, plant extracts and vitamins and vitamin mixtures.

After the ratio of the water phase : the oil phase, and the characteristic α having been determined by the above-mentioned process, the present invention also provides a process for preparing the water-in-oil emulsion system of the present invention according to the above process, which comprises:
(a) mixing uniformly the water phase components, so as to obtain a phase B;
(b) mixing under stirring the oil phase components, the water-in-oil emulsifier and the hydrophobic silica at a speed of 500-2500 rpm, until the hydrophobic silica being dispersed uniformly, so as to obtain the phase A; and
(c) adding the phase B to the phase A at a steady speed for 5-15 minutes under stirring at a speed of 300-1000 rpm, so as to obtain the water-in-oil emulsion system.

As an alternative, the present invention provides a modification of the above preparation process, which process comprises:
(a) mixing uniformly the water phase components, so as to obtain a phase B; and
(b) dividing the oil phase components into two parts, with the first part being mixed uniformly with the emulsifier to obtain a phase A1, and the second part is mixed uniformly with the hydrophobic silica to obtain a phase A2; and
(c) adding gradually the phase B into the phase A1 at a steady speed under stirring at a speed of 300-1000 rpm, so as to obtain a mixture C1, then adding the phase A2 into the mixture C1, and stirring the same uniformly, so as to obtain the water-in-oil emulsion system.

In a preferred embodiment of the process and modification thereof in the present invention, a step (d) is comprised following step (c):
(d) after having completed the addition of all the components in the emulsion system, homogenizing the same for 1-3 minutes by increasing the stirring speed to 1000-10000 rpm.

The present invention will be further described herein below by the embodiments, while these embodiments are by no means to restrict the scope of the present invention.

### Embodiments:

### Embodiments 1-5 and comparative examples 1-2

### Embodiment 1

The steps in determining the optimum weight ratio (characteristic α) of the hydrophobic silica to the water-in-oil emulsifier in the formulation of embodiment 1 are as follows:
(a) it is estimated that in a formulation with a total amount of 100 grams, the water phase accounts for 70wt.% of the total amount, therefore the water phase is determined to be 70 g; and the oil phase accounts for 26,3 wt.% of the total amount, so the oil phase is determined to be 26,3 g, with the oil phase not including hydrophobic silica and the water-in-oil emulsifier; AEROSIL® R812S is used as hydrophobic silica, according to an empirical value (preferably, the hydrophobic silica accounts for 1,5-5 wt.% of the total weight of the emulsion system), the hydrophobic silica is determined to account for 3 wt.% of the total weight, so the amount used is determined to be 3 grams of (X). In addition, ABIL® EM90 is used as a water-in-oil emulsifier. Here, 70 grams of water phase comprised 63,95 grams of deionized water, 5 grams of propanediol, 1 gram of sodium chloride and 0,05 gram of preservative 2-bromo-2-nitro-1,3-propanediol;
(b) 3 grams of AEROSIL® R812S, 26,3 grams of an oil phase (which comprised 1,6 grams of ABIL® WAX9801, 8 grams of TEGO® SOFT DEC, 13,1 grams of cyclic methylsiloxane and 3,6 grams of ABIL® 350) and 0,05 gram (Y₀) of ABIL® EM90 were mixed uniformly, so as to obtain an oil phase mixture A;
(c) 5 grams (Z₀) of water phase were added slowly into the oil phase mixture A under stirring at a speed of 500 rpm, such that the system was in a uniformly emulsified state;
(d) 5 grams (Z₁) of water phase were additionally added, until visually observing that the water phase could no longer be emulsified and, then 0,05 gram (Y₁) of the water-in-oil emulsifier was added into the water-in-oil emulsifier under stirring at a speed of 500 rpm, such that the system was emulsified completely;
(e) step (d) was repeated n times by using Zₙ₊₁ weight parts of the water phase and Yₙ₊₁ weight parts of the water-in-oil emulsifier, until 70 g of water phase was added completely into the oil phase mixture A, and a uniform water-in-oil emulsion system C was obtained, wherein 70= Z₀+Z₁+.....+Zₙ₊₁, and at that time n=13; and
(f) the total weight Y of the water-in-oil emulsifier used in the emulsion system C was calculated as 0,7 gram, wherein Y=Y₀+Y₁+.....+Yₙ₊₁, thereby the optimum weight ratio X/Y of the hydrophobic silica to the water-in-oil emulsifier, i.e. the characteristic ratio α, was obtained. In embodiment 1, the characteristic ratio α = 4,3.

In embodiment 1, the emulsified system finally obtained had a total amount of exactly 100 grams. In the practical operation, in order to determine the characteristic ratio α, if the total amount of the emulsified system finally obtained was more than or less than 100 grams, the ratio that the oil phase accounted for the total amount of the emulsified system, and the ratio that the water phase accounted for the total amount of the emulsified system would have deviations to the expected values; however, the value of the characteristic ratio α was still obtained from the weight ratio of the hydrophobic silica to the water-in-oil emulsifier, and here, the characteristic ratio α was the characteristic ratio of the actual emulsion system but not that of the expected emulsion system.

The water-in-oil emulsion of embodiment 1 was prepared according to the following steps:
(1) 63,95 g of deionized water, 5 g of propanediol, 1 g of NaCl and 0,05 g of a preservative (2-bromo-2-nitro-1,3-propanediol) were mixed under stirring, to obtain a water phase mixture B;
(2) 0,7 g of ABIL®EM90, 1,6g of ABIL® WAX9801, 8 g of TEGO®SOFT DEC and 6,1 g of cyclic methylsiloxane were mixed uniformly under stirring, to obtain an oil phase mixture A1;
(3) 7 g cyclic methylsiloxane, 3,6 g of ABIL® 350 and 3 g of AEROSIL® R812S were mixed uniformly under stirring, to obtain an oil phase mixture A2;
(4) the water phase mixture B was added uniformly into the oil phase mixture A1 under stirring at a speed of 800 rpm for 3 minutes, to obtain a mixture C1;
(5) the oil phase mixture A2 was added into the mixture C1 under stirring at a speed of 800 rpm for 1 minute, to obtain a mixture C2; and
(6) finally the mixture C2 was homogenized under stirring at a speed of 1000 rpm for 3 minutes, to obtain the water-in-oil emulsion system of embodiment 1.

The advantages of the process lie in that when it is used in a large scale production, it can save the preparation time effectively.

### Embodiment 2

The components of phase B shown in Table 1 for embodiment 2 were mixed uniformly under stirring, to obtain the phase B. The components of phase A (here it comprised the oil phase components, the water-in-oil emulsifier and the hydrophobic silica) shown in Table 1 for embodiment 2 were mixed uniformly under stirring at a speed of 800 rpm, until the hydrophobic silica was dispersed uniformly to obtain a phase A. Then, the phase B was added into the phase A under stirring at a speed of 500 rpm for 5 minutes. After completing the addition of the phase B, the stirring speed was increased to 1500 rpm, and then the emulsion obtained was homogenized for 3 minutes, to obtain the water-in-oil emulsion system of embodiment 2.

### Embodiments 3-5

The water-in-oil emulsion systems of embodiments 3-5 were prepared in a way basically similar to that of embodiment 2, except that the phase A component and the phase B component shown for embodiment 2 were replaced by the corresponding phase A component and phase B component shown for embodiments 3-5.

### Comparative examples 1-2

The water-in-oil emulsion systems of comparative examples 1-2 were prepared in a way basically similar to that of embodiment 2, except that the phase A component and the phase B component shown for embodiment 2 were replaced by the corresponding phase A component and the phase B component shown in Table 1 for comparative examples 1-2 .

**Table 1**

| Phase | Ingredient (wt.%) | Embodiment 1 | Embodiment 2 | Embodiment 3 | Embodiment 4 | Embodiment 5 | Comparative example 1 | Comparative example 2 |
|---|---|---|---|---|---|---|---|---|
| A | cetyl PEG/PPG-10/1 polydimethylsiloxa ne (ABIL® EM90) | 0,7 | 1,3 | 1,3 | 0,7 | 0,15 | 0,8 | 0,8 |
| | cetyl polydimethylsiloxane (ABIL® WAX9801) | 1,6 | 1,6 | 1,6 | 1,6 | 1,6 | 1,6 | 1,6 |
| | diethylhexyl carbonate (TEGO® SOFT DEC) | 8 | 8 | 8 | 8 | 8 | 2 | 8 |
| | cyclic methylsiloxane | 13,1 | 12,5 | 12,5 | 13,1 | 13,65 | 3,9 | 10 |
| | polydimethylsiloxane (ABIL® 350) | 3,6 | 3,6 | 3,6 | 3,6 | 3,6 | | 3,6 |
| | microwax | | | | | | | 6 |
| | silylated silica (AEROSIL® R812S) | 3 | | | | | 0,1 | |
| | silylated silica (AEROSIL® R805) | | 3 | | | | | |
| | dimethyl-silylated silica (AEROSIL® R974) | | | 3 | | | | |
| | dimethyl-silylated silica (AEROSIL® R972) | | | | 3 | | | |
| | polydimethylsiloxane silylated silica (AEROSIL® R202) | | | | | 3 | | |
| B | deionized water | 63,95 | 63,9 5 | 63,95 | 63,9 5 | 63,95 | 85,25 | 63,95 |
| | propanediol | 5 | 5 | 5 | 5 | 5 | 5,3 | 5 |
| | sodium chloride | 1 | 1 | 1 | 1 | 1 | 1 | 1 |
| | 2-bromo-2-nitro-1,3-propanediol | 0,05 | 0,05 | 0,05 | 0,05 | 0,05 | 0,05 | 0,05 |
| water-releasing effect | | 5 | 5 | 5 | 5 | 5 | 5 | 1 |
| α | | 4,3 | 2,3 | 2,3 | 4,3 | 20 | | |

The water-releasing effects of the products obtained from embodiments 1-5 and comparative examples 1-2 are represented by a 5-level score.

About 0,2g of a water-in-oil emulsion sample was taken and placed on the back of a hand, then it was applied by circling with the middle finger and ring finger of the other hand gently, and then the phenomenon of the water-releasing effect was observed when the circling application reaches 20 cycles, and evaluated by the 5-level scoring system. Score 5 represented that more than 10 bead-like water drops having an average diameter of more than or equal to 3 mm appeared, or more than 20 bead-like water drops having an average diameter of more than or equal to 1 mm appear; score 4 represented that 2-10 bead-like water drops having an average diameter of more than or equal to 3 mm appeared, or 10-20 bead-like water drops having an average diameter of more than or equal to 1 mm appeared and the bead-like water drops having an average of more than or equal to 3 mm were no more than 10; score 3 represented that 2-9 bead-like water drops having an average diameter of more than or equal to 1 mm appeared and there was at most 1 bead-like water drop having an average diameter of more than or equal to 3 mm, or 10-20 bead-like water drops having an average diameter of 1 mm appeared; score 2 represented that 2-9 bead-like water drops having an average diameter of 1 mm appeared; and score 1 represented that no water drop appeared. Each level between scores 5 to 4, 4 to 3, 3 to 2, 2 to 1 showed that the water-releasing effect was between the two end values described above, and the lower the score, the poorer the water-releasing effect. A water-releasing effect having an evaluation result of more than or equal to score 3 was defined as a "good water-releasing effect" of the present invention. The water-releasing effects of embodiments 1-5 and comparative examples 1-2 are shown in Table 1.

Embodiments 1-5 were the cases that when the content of the internal phase (water phase) was fixed to about 70 wt.%, the ratios of the emulsifier of ABIL® EM90 and various hydrophobic silica products were changed. With the different types of hydrophobic fumed silica, the optimum amounts of ABIL® EM90 used were also different, thus the optimum water-releasing effects could be achieved.

The formulation of comparative example 1 was a conventional water-in-oil formulation, which included a small amount of hydrophobic silica (0,1 wt.%), and a good water-releasing effect was achieved only when the total amount of water and propanediol in the internal phase (water phase) reached about 90 wt.%.

In view of the influences of the viscosity to the water-releasing effect, the present inventor designed a comparative example 2, the formulation of which was very similar to those of the embodiments 1-5, except that hydrophobic silica was replaced by microcrystalline wax of 6 wt.% to obtain a viscosity close to those of the embodiments 1-5, but the product obtained by this formulation had no water-releasing effect. This has demonstrated that in the present invention the hydrophobic fumed silica does not merely function as a conventional thickener. The synergy between the hydrophobic silica and the emulsifier give the common water-in-oil system of a low content of water phase a good water-releasing effect.

### Embodiments 6-8

The water-in-oil emulsion systems of embodiments 6-8 were prepared in a way basically similar to that of embodiment 2, except that the phase A component and the phase B component shown for embodiment 2 were replaced by the corresponding phase A component and the phase B component shown in Table 2 respectively for embodiments 6-8. A water-releasing effect test was carried out with the products prepared in the same way as that in embodiment 1, and the results were shown in Table 2.

**Table 2**

| Phase | Ingredient (wt.%) | Embodiment 6 | Embodiment 7 | Embodiment 8 |
|---|---|---|---|---|
| A | cetyl PEG/PPG-10/1 polydimethylsiloxane (ABIL® EM90) | 1,1 | 0,7 | 0,4 |
| | cetyl polydimethylsiloxane (ABIL® WAX9801) | 1,6 | 1,6 | 1,6 |
| | diethylhexyl carbonate (TEGO® SOFT DEC) | 8 | 8 | 8 |
| | cyclic methylsiloxane | 25,7 | 22,6 | 4,9 |
| | polydimethylsiloxane (ABIL® 350) | 3,6 | 3,6 | 3,6 |
| | silylated silica (AEROSIL® R812S) | 5 | 3,5 | 1,5 |
| B | deionized water | 48,95 | 53,95 | 73,95 |
| | propanediol | 5 | 5 | 5 |
| | sodium chloride | 1 | 1 | 1 |
| | preservative | 0,05 | 0,05 | 0,05 |
| water-releasing effect | | 4 | 4,5 | 5 |
| α | | 4,5 | 5 | 3,75 |

For the water-in-oil emulsion systems prepared in embodiments 6-8, a combination of ABIL® EM90 and AEROSIL® R812S was used, and the water phase contents were respectively 55 wt.%, 60 wt.% and 80 wt.%.

### Embodiments 9-13

Embodiments 9-13 showed the combinations of ISOLAN® PDI with various hydrophobic fumed phase silica.

The water-in-oil emulsion systems of embodiments 9-13 were prepared in a way basically similar to that of embodiment 2, except that the phase A component and the phase B component shown for embodiment 2 were replaced by the corresponding phase A component and phase B component shown for embodiments 9-13. A water-releasing effect test was carried out with the products prepared in the same way as that in embodiment 1, and the results were shown in Table 3.

**Table 3**

| Phase | Ingredient (wt.%) | Embodiment 9 | Embodiment 10 | Embodiment 11 | Embodiment 12 | Embodiment 13 |
|---|---|---|---|---|---|---|
| A | diisostearyl polyglyceryl-3 dimer dilinoleate (ISOLAN® PDI) | 0,2 | 0,2 | 0,4 | 0,25 | 0,25 |
| | cetyl polydimethylsiloxane (ABIL® WAX9801) | 1,6 | 1,6 | 1,6 | 1,6 | 1,6 |
| | diethylhexyl carbonate (TEGO® SOFT DEC) | 8 | 8 | 8 | 8 | 8 |
| | cyclic methylsiloxane | 13,6 | 13,6 | 13,4 | 13,55 | 13,55 |
| | white mineral oil | 3,6 | 3,6 | 3,6 | 3,6 | 3,6 |
| | silylated silica (AEROSIL® R812S) | | | | | 3 |
| | silylated silica (AEROSIL® R805) | | 3 | | | |
| | dimethyl-silylated silica (AEROSIL® R974) | | | 3 | | |
| | dimethyl-silylated silica (AEROSIL® R972) | | | | 3 | |
| | polydimethylsiloxane silylated silica (AEROSIL® R202) | 3 | | | | |
| B | deionized water | 63,95 | 63,95 | 63,95 | 63,95 | 63,95 |
| | propanediol | 5 | 5 | 5 | 5 | 5 |
| | sodium chloride | 1 | 1 | 1 | 1 | 1 |
| | preservative | 0,05 | 0,05 | 0,05 | 0,05 | 0,05 |
| water-releasing effect | | 5 | 5 | 5 | 5 | 5 |
| α | | 15 | 15 | 7,5 | 12 | 12 |

### Embodiments 14-18

Embodiments 14-18 showed the combinations of ISOLAN® GPS with various hydrophobic fumed phase silica.

The water-in-oil emulsion systems of embodiments 14-18 were prepared in a way basically similar to that of embodiment 2, except that the phase A component and the phase B component shown for embodiment 2 were replaced by the corresponding phase A component and the phase B component shown in Table 4 for embodiments 14-18. A water-releasing effect test was carried out with the products prepared in the same way as that in embodiment 1, and the results were shown in Table 4.

**Table 4**

| Phase | Ingredient (wt.%) | Embodiment 14 | Embodiment 15 | Embodiment 16 | Embodiment 17 | Embodiment 18 |
|---|---|---|---|---|---|---|
| A | polyglyceryl-4 diisostearate/polyhy droxystearate/sebaca te (ISOLAN® GPS) | 0,1 | 0,5 | 0,55 | 0,25 | 0,2 |
| | cetyl polydimethylsiloxane (ABIL® WAX9801) | 1,6 | 1,6 | 1,6 | 1,6 | 1,6 |
| | diethylhexyl carbonate (TEGO® SOFT DEC) | 8 | 8 | 8 | 8 | 8 |
| | cyclic methylsiloxane | 13,7 | 13,3 | 13,25 | 13,55 | 13,6 |
| | white mineral oil | 3,6 | 3,6 | 3,6 | 3,6 | 3,6 |
| | silylated silica (AEROSIL® R812S) | | | | | 3 |
| | silylated silica (AEROSIL® R805) | | 3 | | | |
| | dimethyl-silylated silica (AEROSIL® R974) | | | 3 | | |
| | dimethyl-silylated silica (AEROSIL® R972) | | | | 3 | |
| | polydimethylsiloxane silylated silica (AEROSIL® R202) | 3 | | | | |
| B | deionized water | 63,95 | 63,95 | 63,95 | 63,95 | 63,95 |
| | propanediol | 5 | 5 | 5 | 5 | 5 |
| | sodium chloride | 1 | 1 | 1 | 1 | 1 |
| | preservative | 0,05 | 0,05 | 0,05 | 0,05 | 0,05 |
| water-releasing effect | | 5 | 5 | 5 | 5 | 5 |
| α | | 30 | 6 | 5,5 | 12 | 15 |

### Embodiments 19-23

Embodiments 19-23 showed the combinations of ABIL® EM97 with various hydrophobic fumed phase silica.

The water-in-oil emulsion systems of embodiments 19-23 were prepared in a way basically similar to that of embodiment 2, except that the phase A component and the phase B component shown for embodiment 2 were replaced by the corresponding phase A component and phase B component shown in Table 5 for embodiments 19-23. A water-releasing effect test was carried out with the products prepared in the same way as that in embodiment 1, and the results were shown in Table 5.

**Table 5**

| Phase | Ingredient (wt.%) | Embodiment 19 | Embodiment 20 | Embodiment 21 | Embodiment 22 | Embodiment 23 |
|---|---|---|---|---|---|---|
| A | bis-PEG/PPG-14/14polydimethylsilo xane (ABIL® EM97) | 0,4 | 1,15 | 1,95 | 1,1 | 1 |
| | cetyl polydimethylsiloxane (ABIL® WAX9801) | 1,6 | 1,6 | 1,6 | 1,6 | 1,6 |
| | cyclic methylsiloxane | 21,4 | 20,65 | 19,85 | 20,7 | 20,8 |
| | polydimethylsiloxane (ABIL® 350) | 3,6 | 3,6 | 3,6 | 3,6 | 3,6 |
| | silylated silica (AEROSIL® R812S) | | | | | 3 |
| | silylated silica (AEROSIL® R805) | | 3 | | | |
| | dimethyl-silylated silica (AEROSIL® R974) | | | 3 | | |
| | dimethyl-silylated silica (AEROSIL® R972) | | | | 3 | |
| | polydimethylsiloxane silylated silica (AEROSIL® R202) | 3 | | | | |
| B | deionized water | 63,95 | 63,95 | 63,95 | 63,95 | 63,95 |
| | propanediol | 5 | 5 | 5 | 5 | 5 |
| | sodium chloride | 1 | 1 | 1 | 1 | 1 |
| | preservative | 0,05 | 0,05 | 0,05 | 0,05 | 0,05 |
| water-releasing effect | | 5 | 5 | 5 | 5 | 5 |
| α | | 7,5 | 2,6 | 1,5 | 2,7 | 3 |

### Embodiment 24

Each component of B phase shown in Table 6 was mixed uniformly under stirring, to obtain a phase B. Each component of phase A (here it comprised the oil phase components, the water-in-oil emulsifier and the hydrophobic silica) shown in Table 6 was mixed uniformly under stirring at a speed of 1000 rpm for 10 minutes, to disperse AEROSIL® R974, so as to obtain a phase A. Then, the phase B was added slowly into the phase A under stirring at a speed of 500 rpm. After completing the addition of the phase B, the stirring speed was increased to 1300 rpm, and then the emulsion obtained was homogenized for 3 minutes, to obtain the water-in-oil emulsion system. A water-releasing effect test was carried out with the products prepared in the same way as that in embodiment 1, and the results were shown in Table 6.

**Table 6**

| Phase | | (wt.%) | (wt.%) |
|---|---|---|---|
| A | cetyl PEG/PPG-10/1 polydimethylsiloxane (ABIL® EM90) | 0,71 | 24 |
| | cetyl polydimethylsiloxane (ABIL® WAX9801) | 1 | |
| | cyclic methylsiloxane /dimethylsilanol (ABIL® OSW5) | 5 | |
| | diethylhexyl carbonate (TEGO® SOFT DEC) | 15,79 | |
| | dimethyl-silylated silica (AEROSIL® R974) | 1,5 | |
| B | water | 69,5 | 76 |
| | NaCl | 1 | |
| | glycerol | 5 | |
| | DMDMH (1,3-dihydroxymethyl-5,5-dimethyl hydantoin ) | 0,5 | |
| water-releasing effect | | 5 | |
| α | | 2,1 | |

In embodiment 24, an oil phase of a high viscosity was used (cyclic methylsiloxane of a low viscosity was not used); at that time, the content of the hydrophobic fumed silica was reduced properly (reducing from 3 wt.% to 1,5 wt.%), the amount of the emulsifier ABIL® EM90 was adjusted according to a ratio relationship (the optimum ratio of AEROSIL® R974 to ABIL® EM90 was between 2,1 to 2,3), and the water-releasing effect was still very good. At that time, the hydrophobic fumed silica (not including the hydrophobic silica and water-in oil emulsifier) accounted for 7 wt.% of the oil phase.

If an oil phase of a higher viscosity was used and the amount of the hydrophobic fumed silica used was further reduced, for example, reduce the amount of the hydrophobic fumed silica to less than 5 wt.% of the oil phase (not including the hydrophobic silica and water-in-oil emulsifier) and adjust the amount of the emulsifier used according to a characteristic ratio α, this mixture would not be able to form a stable emulsified system because of the too small amount of the emulsifier.

### Embodiment 25

The water-in-oil emulsion system of embodiment 25 was prepared in a way basically similar to that of embodiment 24, except that the phase A component and the phase B component shown for embodiment 24 were replaced by the corresponding phase A component and phase B component shown in Table 7 for embodiments 25. A water-releasing effect test was carried out with the products prepared in the same way as that in embodiment 1, and the results were shown in Table 7.

**Table 7**

| Phase | | (wt.%) | (wt.%) |
|---|---|---|---|
| A | cetyl PEG/PPG-10/1 polydimethylsiloxane (ABIL® EM90) | 0,71 | 40 |
| | cetyl polydimethylsiloxane (ABIL® WAX9801) | 1 | |
| | cyclic methylsiloxane /dimethylsilanol (ABIL® OSW5) | 5 | |
| | diethylhexyl carbonate (TEGO® SOFT DEC) | 31,79 | |
| | dimethyl-silylated silica (AEROSIL® R974) | 1,5 | |
| B | water | 53,5 | 60 |
| | NaCl | 1 | |
| | glycerol | 5 | |
| | DMDMH (1,3-dihydroxymethyl-5,5-dimethyl hydantoin ) | 0,5 | |
| water-releasing effect | | 4 | |
| α | | 2,1 | |

### Embodiment 26

The water-in-oil emulsion system of embodiments 26 was prepared in a way basically similar to that of embodiment 6, except that the phase A component and the phase B component shown for embodiment 6 were replaced by the corresponding phase A component and the phase B component shown in Table 8 for embodiments 26. A water-releasing effect test was carried out with the products prepared in the same way as that in embodiment 1, and the results were shown in Table 8.

**Table 8**

| Phase | Ingredient (wt.%) | (wt.%) | (wt.%) |
|---|---|---|---|
| A | cetyl PEG/PPG-10/1 polydimethylsiloxane (ABIL® EM90) | 1,1 | 35 |
| | cetyl polydimethylsiloxane(ABIL® WAX9801) | 1,6 | |
| | diethylhexyl carbonate (TEGO® SOFT DEC) | 8 | |
| | cyclic methylsiloxane | 15,7 | |
| | polydimethylsiloxane (ABIL® 350) | 3,6 | |
| | silylated silica (AEROSIL® R812S) | 5 | |
| B | deionized water | 58,95 | 65 |
| | propanediol | 5 | |
| | sodium chloride | 1 | |
| | preservative | 0,05 | |
| water-releasing effect | | 3 | |
| α | | 4,5 | |

### Embodiment 27

Each component of B phase shown in Table 9 for embodiment 27 was mixed uniformly under stirring, to obtain a phase B. Each component of A phase (here it comprised the oil phase components, the water-in-oil emulsifier ABIL® EM90 and the hydrophobic precipitated silica SIPERNAT® D17) shown in Table 9 for embodiment 27 was mixed uniformly under stirring at a speed of 800 rpm, until SIPERNAT® D17 was dispersed uniformly, thus obtaining a phase A. Then, the phase B was added at a steady speed into phase A under stirring at a speed of 500 rpm for 5 minutes. After completing the addition of the phase B, the stirring speed was increased to 1500 rpm, and then the emulsion obtained was homogenized for 3 minutes, thus obtaining the water-in-oil emulsion system.

**Table 9**

| Phase | | (wt.%) |
|---|---|---|
| A | cetyl PEG/PPG-10/1 polydimethylsiloxane (ABIL® EM90) | 0,4 |
| | hexadecyldimethylsiloxane (ABIL® Wax9801) | 1,6 |
| | diethylhexyl carbonate (TEGO®SOFT DEC) | 8 |
| | cyclic methylsiloxane (DC 345) | 13,4 |
| | polydimethylsiloxane (ABIL® 350) | 3,6 |
| | hydrophobic precipitation silica (SIPERNAT® D17) | 3 |
| B | water | 53,95 |
| | glycerol | 15 |
| | NaCl | 1 |
| | preservative | 0,05 |
| water-releasing effect | | 5 |
| α | | 7,5 |

Each water-in-oil system prepared in embodiments 1-27 was respectively subjected to a stability test at room temperature, a thermal resistance test and a freezing and thawing test. The stability test at room temperature was referred to that the sample was stored for three months at room temperature between 20-25°C; the thermal resistance test was referred to that the sample was stored for three months at 45°C; and the freezing and thawing test was referred to that the sample was subjected to three cold/hot cycles at -15°C/room temperature. As a result, after having been subjected to the above tests, the appearances of these products without one exception, remained to be uniform and no phenomena of discoloration, delamination, etc. occurred. It was shown thereby that the water-in-oil emulsion system of the present invention was stable.

## Claims

1. A water-in-oil emulsion system, comprising, on the basis of the total weight of the emulsion system,
1,5-9 wt.% of hydrophobic silica,
0,1-5 wt.% of a water-in-oil emulsifier,
10-40 wt.% of an oil phase, with the content of the oil phase not including said hydrophobic silica and water-in-oil emulsifier, and
55-85 wt.% of a water phase,
with the water-in-oil emulsion system having a good water-releasing effect,
with the proviso that said hydrophobic silica is not AEROSIL® RY200 hydrophobic fumed silica, and it is excluded the formulation comprising the components of: polyoxyalkylene modified polysiloxane SH3775E (produced by Toray Silicone company), an alkyl glyceryl ether modified polysiloxane of the following formula (I) and hydrophobic fumed silica AEROSIL® R812 (produced by Degussa company), in which the weight ratio of SH3775E to said alkyl glyceryl ether modified polysiloxane is 0,5, and the weight ratio of the sum of SH3775E and said alkyl glyceryl ether modified polysiloxane to said hydrophobic fumed silica AEROSIL® R812 is also 0,5, 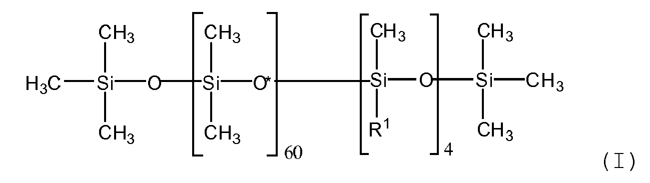 in which

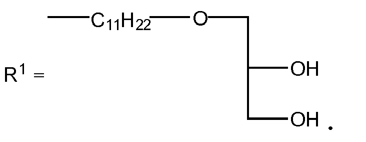

2. A water-in-oil emulsion system, comprising, on the basis of the total weight of the emulsion system,
1,5-9 wt.% of hydrophobic silica,
0,1-5 wt.% of a water-in-oil emulsifier,
10-40 wt.% of an oil phase, with the content of the oil phase not including hydrophobic silica and water-in-oil emulsifier, and
55-85 wt.% of a water phase,
wherein the weight ratio of the hydrophobic silica to the oil-in-water emulsifier is preferably in a range between 0,7α to 1,2α, in which α is the optimum ratio of the hydrophobic silica to the oil-in-water emulsifier, which is also referred to as a characteristic ratio α, and which is obtained by the process of:
(i) providing the weight parts of the hydrophobic silica, the oil phase and the water phase in the water-in-oil emulsion respectively as weight parts X, M, and Z, wherein the oil phase does not include hydrophobic silica and the water-in-oil emulsifier;
(ii) mixing uniformly X weight parts of the hydrophobic silica, M weight parts of the oil phase, Y₀ weight parts of the water-in-oil system, so as to obtain an oil phase mixture A;
(iii) adding slowly under stirring Z₀ weight parts of the water phase into the oil phase mixture A, so as to make the system into a uniformly emulsified state;
(iv) adding continuously Z₁ weight parts of the water phase, until visually observing that the water phase can no longer be emulsified, and then adding under stirring Y₁ weight parts of the water-in-oil emulsifier into the emulsion system, so as to have the system fully emulsified;
(v) repeating step (iv) n times by using Zₙ₊₁ weight parts of the water phase and Yₙ₊₁ weight parts of the water-in-oil emulsifier, until all of Z weight parts of the water phase having been added to the oil phase mixture A, thus obtaining a uniform water-in-oil emulsion system C, wherein Z=Z₀ + Z₁ +.....+ Zₙ₊₁, and wherein n is a natural number and n>1; and
(vi) calculating the total weight Y of the water-in-oil emulsifier used in the emulsion system C, wherein Y=Y₀+Y₁+.....+Yₙ₊₁, thereby obtaining the optimum weight ratio X/Y of the hydrophobic silica to the water-in-oil emulsifier, i.e. the characteristic ratio α, in which n is a natural number, and n>1,
with the proviso that said hydrophobic silica is not AEROSIL® RY200 hydrophobic fumed silica, and it is excluded the formulation comprising the components of: polyoxyalkylene modified polysiloxane SH3775E (produced by Toray Silicone company), an alkyl glyceryl ether modified polysiloxane of the following formula (I) and hydrophobic fumed silica AEROSIL® R812 (produced by Degussa company), in which the weight ratio of SH3775E to the alkyl glyceryl ether modified polysiloxane is 0,5, and the weight ratio of the sum of SH3775E and the alkyl glyceryl ether modified polysiloxane to hydrophobic fumed silica AEROSIL® R812 is also 0,5, 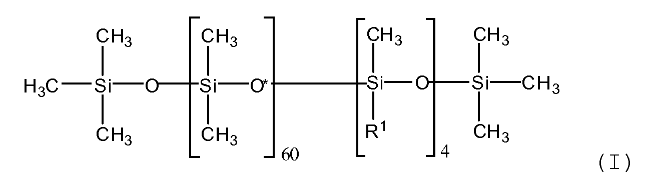 in which

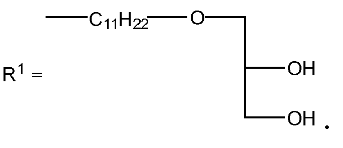

3. The emulsion system as claimed in claim 2, wherein Y₀, Y₁.....Yₙ₊₁ weight parts of the water-in-oil emulsifier can be either the same or different in terms of weight, and each of them is made such that its content is 0,01-0,3 wt.%, and preferably 0,05-0,2 wt.%, based on the total weight of the water-in-oil emulsion system; and Z₀, Z₁.....Zₙ₊₁ weight parts of the water phase can be either the same or different in terms of weight, and each of them is made such that its content is 2-9 wt.%, and preferably 4-9 wt.%, based on the total weight of the water-in-oil emulsion system.

4. The emulsion system as claimed in any of claims 1-3, wherein the hydrophobic silica accounts for 5-15 wt.% of the weight of the oil phase, preferably 5,5-15 wt.%, more preferably 5,5-13 wt.%, and particularly 7-13 wt.%; and the oil phase herein does not include the hydrophobic silica and the water-in-oil emulsifier.

5. The emulsion system as claimed in any of claims 1-4, comprising, on the basis of the total weight of the emulsion system,
1,5-5 wt.% of the hydrophobic silica,
0,3-2,5 wt.% of the water-in-oil emulsifier,
15-40 wt.% of the oil phase, with the content of the oil phase not including the above hydrophobic silica and the water-in-oil emulsifier, and
55-80 wt.% of the water phase.

6. The emulsion system as claimed in any of claims 1-5, wherein the hydrophobic silica is hydrophobic fumed silica, and particularly those of a methanol wettability (hydrophobicity) of 10-85%, and preferably 25-75%, and having a BET surface area between 70-350 m²/g, and preferably 100-280 m²/g; preferably the hydrophobic silica is one or more selected from the group consisting of AEROSIL® R202, AEROSIL® R972, AEROSIL® R805, AEROSIL® R8200, AEROSIL® R974, AEROSIL® R812S, AEROSIL® R812 and SIPERNAT® D17.

7. The emulsion system as claimed in any of claims 1-6, wherein the water-in-oil emulsifier is a surfactant having an HLB value of 2-8.

8. The emulsion system as claimed in any of claims 1-6, wherein the following combinations of the hydrophobic silica and the water-in-oil emulsifier are used, and the preferred characteristic ratio α of these combinations are shown in the following table:
| characteristic | ABIL® | ISOLAN® | ISOLAN® | ABIL® |
|---|---|---|---|---|
| ratio α | EM90 | PDI | GPS | EM97 |
| AEROSIL® R812S | 4,3 | 12 | 15 | 3 |
| AEROSIL® R805 | 2,3 | 15 | 6 | 2,6 |
| AEROSIL® R974 | 2,3 | 7,5 | 5,5 | 1,5 |
| AEROSIL® R972 | 4,3 | 12 | 12 | 2,7 |
| AEROSIL® R202 | 20 | 15 | 30 | 7,5 |
| SIPERNAT® D17 | 7,5 | | | |

9. A process for preparing the water-in-oil emulsion system as claimed in any of claims 1-8, comprising:
(a) mixing uniformly the water phase components, so as to obtain the phase B;
(b) mixing under stirring the oil phase components, the water-in-oil emulsifier and the hydrophobic silica at a speed of 500-2500 rpm, until the hydrophobic silica being dispersed uniformly, so as to obtain the phase A; and
(c) adding the phase B to the phase A at a steady speed for 5-15 minutes under stirring at a speed of 300-1000 rpm, so as to obtain the water-in-oil emulsion system.

10. A process for preparing the water-in-oil emulsion system as claimed in any of claims 1-8, comprising:
(a) mixing uniformly the water phase components, so as to obtain a phase B; and
(b) dividing the oil phase components into two parts, with the first part being mixed uniformly with the emulsifier to obtain a phase A1, and the second part being mixed uniformly with the hydrophobic silica to obtain a phase A2; and
(c) adding gradually the phase B into the phase A1 at a steady speed under stirring at a speed of 300-1000 rpm, so as to obtain a mixture C1, then adding the phase A2 into the mixture C1, and stirring the same uniformly, so as to obtain the water-in-oil emulsion system.

11. The process as claimed in claim 9 or 10, wherein it further comprises a step (d) following the step (c):
(d) after having completed the addition of all the components in the emulsion system, homogenizing the same for 1-3 minutes by increasing the stirring speed to 1000-10000 rpm.
